# EUROPEAN PATENT SPECIFICATION

(11) **EP 3 096 724 B1**
(45) Date of publication and mention of the grant of the patent: **11.11.2020**
(21) Application number: 15713321.6
(22) Date of filing: 26.03.2015
(51) Int. Cl.: A61F 9/007

(54) **METHOD OF MANUFACTURING AN OPHTHALMIC SURGICAL INSTRUMENT AND OPHTHALMIC SURGICAL INSTRUMENT**
VERFAHREN ZUR HERSTELLUNG EINES OPHTHALMISCH-CHIRURGISCHEN INSTRUMENTES UND OPHTHALMISCH-CHIRURGISCHES INSTRUMENT
MÉTHODE DE FABRICATION D'UN INSTRUMENT DE CHIRURGIE OCULAIRE ET INSTRUMENT DE CHIRURGIE OCULAIRE

(30) Priority: 02.05.2014 US 201414268321
(43) Date of publication of application: 30.11.2016
(73) Proprietor: Alcon Inc., 1701 Fribourg (CH)
(72) Inventor: SCHALLER, Philipp, CH-8203 Schaffhausen (CH)
(74) Representative: Hanna Moore + Curley
(86) International application number: PCT/EP2015/056641
(87) International publication number: WO 2015/165666

(56) References cited:
- WO-A2-2007/047030
- US-A- 3 656 481
- US-A1- 2005 054 953
- US-A1- 2005 090 849
- US-A1- 2005 234 473

## Description

### TECHNICAL FIELD

Embodiments disclosed herein are related to a method of manufacturing an ophthalmic surgical instrument and to an ophthalmic surgical instrument manufactured with said method. More specifically, embodiments described herein relate to an instrument tip with an internal frame defining an interior structure and an external coating defining an exterior surface.

### BACKGROUND

Ophthalmic surgical instruments can include complex distal tips that are used by a surgeon to manipulate a patient's anatomy (e.g., one or more layers of the patient's eye). The designs of these instrument tips can be a combination of free formed surfaces, defined by small edge radii and other demanding design features. Conventionally, the instrument tips are manufactured by hand, using manual processes. For example, the instrument tips can be hand-rolled, bent, grinded, polished, etc., to finish and ensure the surface is smooth and free of edges or burrs. Manufacturing can thus be highly laborious and time-consuming.

The instrument tips are conventionally manufactured using entirely metal (e.g., stainless steel) to ensure an appropriate bending stiffness for the component. Further, the metallic instrument tip can directly contact the patient's anatomy. The materials used can thus have demanding technical requirements (e.g., flexibility, stiffness, porosity, hardness, density, etc.), resulting in high monetary expense. Because of the high material and manufacturing costs in money and time, it is not economically feasible to make the instrument tips disposable or single-use.

Microsurgical instruments are additionally difficult to manufacture by hand, while satisfying the necessary technical requirements, because they are extremely small. For example, in flapless refractive surgery, an ophthalmic surgical instrument, the lenticule manipulator, can be used to delaminate the lenticule after treatment of the cornea by UV Femtolaser. This surgical instrument can be characterized by the complex shape of its tip, which is used to manipulate individual layers of the eye. Other ophthalmic surgical instruments can similarly include complex tip designs with high technical requirements.

US2005090849 relates to an angled tissue cutting instrument comprises an elongate angled outer tubular member and an elongate flexible inner tubular member rotatably disposed within the outer member to transmit torque to a cutting configuration of the inner member when the inner member is rotated relative to and within the outer member in forward and reverse rotational directions. The inner member comprises an inner tube, a helical cut formed along a length portion of the inner tube to impart flexibility and no more than a single layer of spiral wrap disposed over the helically cut length portion of the inner tube. A method of fabricating an angled tissue cutting instrument involves forming the helical cut in an inner tube having an inner diameter the same size as the inner diameter of the straight inner tubular member of a straight tissue cutting instrument of the same diametric size as the angled tissue cutting instrument. US2005/0234473 relates to surgical probe with a tip shield for lens removal by vibratory surgical instruments. The probe with tip shield including a tip cap disposed as a membrane covering the distal end surface of a vibratory probe to act to protect the lens capsule during operation. Direct contact between the metallic vibratory probe and the lens capsule is avoided by the interposed tip cap even when the capsule is aspirated by the central aspiration opening. US 3 656 481 discloses another ophthalmic surgical instrument.

### SUMMARY

It will be appreciated that the scope of the invention is in accordance with the claims. Accordingly, there is provided a method of manufacturing an ophthalmic surgical instrument as defined in claim 1. Also provided is an ophthalmic surgical instrument manufactured according to the method of invention in accordance with claim 7. Further features are provided in the dependent claims. It is noted that the specification may also include description of arrangements outside the scope of the claims but provided as background and to assist in understanding the invention.

The presented solution fills an unmet medical need with a unique solution to provide ophthalmic surgical instrument tips with an internal frame and an external coating that can be manufactured faster and more cost- effectively while providing the ability to generate complex shapes and meet necessary technical requirements.

Consistent with some embodiments. an ophthalmic surgical instrument comprises: an instrument body having a proximal end, a distal end, and a longitudinal axis; and an instrument tip disposed at the distal end of the instrument body, the instrument tip including a first section linearly extending along a longitudinal axis of the instrument body and a second section extending obliquely from the first section, the second section being arcuately shaped; and wherein the instrument tip comprises a first internal frame and a first coating covering the first internal frame.

Consistent with some embodiments, a method of manufacturing an ophthalmic surgical instrument comprises: generating an internal frame defining an internal structure of an instrument tip, the instrument tip having a distal end, a linear first section, an arcuate second section extending obliquely from the first section, and a flattened portion at the distal end; generating an instrument body using a coating, the instrument body having a longitudinal axis and an external surface of the instrument body including surface features; and covering the internal frame with the coating to define an exterior surface of the instrument tip simultaneously as generating the instrument body, wherein the cross-sectional profiles of the internal frame and coating in a plane perpendicular to the longitudinal axis of the instrument body define different shapes along at least a portion of the longitudinal axis.

Additional aspects, features, and advantages of the present disclosure will become apparent from the following detailed description.

### BRIEF DESCRIPTION OF THE DRAWINGS

**FIG. 1** is a flow diagram illustrating a method of manufacturing an ophthalmic surgical instrument.
**FIG. 2** is a flow diagram illustrating a method of manufacturing an ophthalmic surgical instrument, this method not being part of the claimed invention.
**FIG. 3** is a flow diagram illustrating a method of manufacturing an ophthalmic surgical instrument, this method not being part of the claimed invention.
**FIG. 4** is a diagram illustrating an internal frame of an instrument tip of an ophthalmic surgical instrument.
**FIG. 5a** is a diagram illustrating an instrument tip of an ophthalmic surgical instrument.
**FIG. 5b** is a diagram illustrating an instrument tip of an ophthalmic surgical instrument.
**FIG. 6** is a diagram illustrating a cross-sectional view of an instrument tip of an ophthalmic surgical instrument.
**FIG. 7a** is a diagram illustrating an instrument body of an ophthalmic surgical instrument, this instrument not being part of the claimed invention.
**FIG. 7b** is a diagram illustrating an internal frame of an instrument body of an ophthalmic surgical instrument, this instrument not being part of the claimed invention.
**FIG. 7c** is a diagram illustrating an instrument body of an ophthalmic surgical instrument, this instrument not being part of the claimed invention.
FIG. 8 is a diagram illustrating a unified internal frame of an instrument tip and an instrument body of an ophthalmic surgical instrument, this instrument not being part of the claimed invention.
Fig. 9a is a diagram illustrating an ophthalmic surgical instrument.
FIG. 9b is a diagram illustrating an ophthalmic surgical instrument, this instrument not being part of the claimed invention.

In the drawings, elements having the same designation have the same or similar functions.

### DETAILED DESCRIPTION

In the following description specific details are set forth describing certain arrangements. It will be apparent, however, to one skilled in the art that the disclosed arrangements may be practiced without some or all of these specific details..

The present disclosure describes an ophthalmic surgical instrument tip with a metal skeleton or an internal frame that is sheathed with plastic or a coating. The internal frame can ensure the technical requirements for flexibility and/or stiffness are satisfied, while the complex outer profile of the instrument tip can be defined by the coating. Instruments manufactured according to the present disclosure can be used for refractive surgery, cataract surgery, vitreoretinal surgery, and/or other ophthalmic surgical procedures.

The internal frame can be manufactured using a relatively inexpensive component, such as a blanked, wire eroded, or metal injection molded (MIM) metal part. The internal frame can be used as an insert in an injection molding process, which covers the internal frame with the coating to define the outer profile. This present disclosure describes the manufacture of instruments with comparable technical properties as hand-made instruments but with lower materials and manufacturing costs. Thus, disposable or single-use instruments are more feasible to manufacture.

The devices, systems, and methods of the present disclosure provide numerous advantages, including: (1) faster, less laborious, and more cost-effective manufacturing based on relatively simple materials/industrial processing compared to manual processing; (2) more cost-effective materials such as lower cost bulk goods with less stringent technical requirements; (3) unlimited design complexity using injection molding; (4) high process stability using established materials/industrial processing (die-cutting, bending, injection molding, etc.); (6) flexibility in manufacturing multiple components of the instrument, such as the body and the tip, using the same processing steps; (7) automation of manufacturing using established materials/industrial processing; and (8) flexibility in choosing internal frame and external coating materials based on surgical objectives.

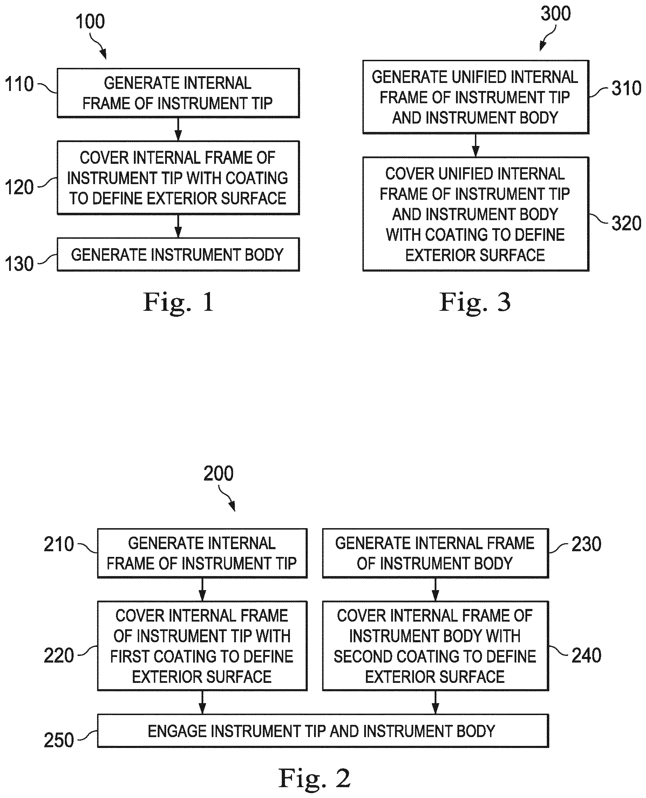

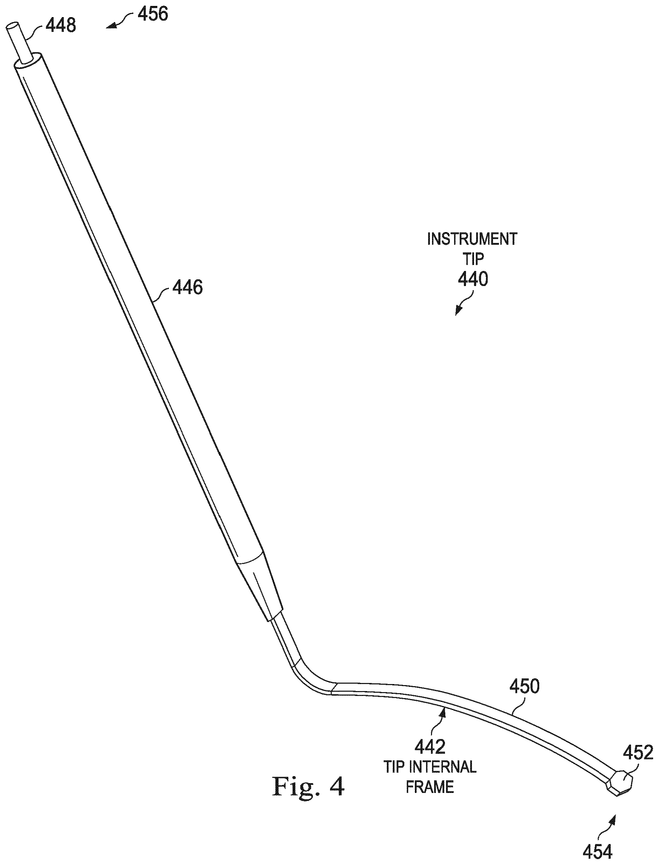

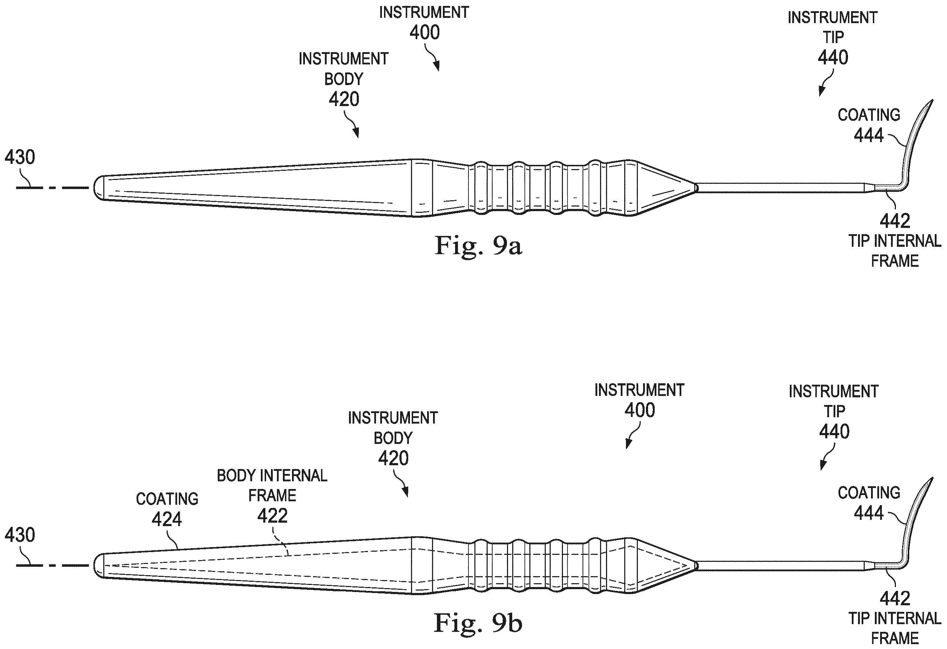

**FIGS. 1-3** provide flow diagrams of methods 100, 200, and 300, respectively, of manufacturing an ophthalmic surgical instrument 400 **(****FIGS. 9a** and **9b**). The methods 100, 200, and 300, can be further understood with reference to **FIGS. 4-9b****,** which illustrate the instrument 400 in various stages of the methods 100, 200, and 300. **FIGS. 9a** and **9b** can illustrate the fully-assembled instrument 400. The instrument 400 includes an instrument body 420 and an instrument tip 440. The method 100 describes the manufacture of an instrument 400 in which the instrument tip 440, and not the instrument body 420, includes an internal frame and an external coating. The methods 100 and 200 can describe the manufacture of an instrument 400 in which the instrument body 420 and the instrument tip 440 each include an internal frame and an external coating. In the method 100, the instrument body 420 and the instrument tip 440 are separate components, while in the method 200, the instrument body and the instrument tip are a single component.

Referring to **FIG. 1****,** the method 100 includes, at step 110, generating an internal frame of an instrument tip. The method 100 further includes, at step 120, covering the tip internal frame with a first coating to define an exterior surface of the instrument tip. As shown in **FIGS. 4-6** and **8-9b**, the instrument tip 440 is sized and shaped to contact one or more layers of the patient's eye during a surgical procedure. The tip internal frame 442 defines an interior structure of the instrument tip 440. The coating 444 defines the exterior surface of the instrument tip 440. Using the coating to define the exterior surface can allow for a complex tip shape to be generated in a faster and more cost efficient manner (compared to, e.g., rolling, bending, filing and polishing a wholly metallic instrument tip 440). At the same time, the tip internal frame 442 can provide necessary flexibility and/or stiffness for the instrument tip 440 as the coating 444 is in contact with the patient's anatomy during a surgical procedure. Providing the necessary flexibility and/or stiffness can allow movements of a surgeon's hand to be translated to the instrument tip. Additional flexibility and/or stiffness are provided by the coating 444. The coating 444 can also be selected to satisfy various technical requirements including, for example, surface roughness, surface structure, porosity, hardness, density, etc.

The tip internal frame 442 can be made of or include a first material, such as a metal, a metal alloy, a ceramic, a composite, a polymer, a plastic, an elastomer, and/or any other suitable material. The first material can be selected such that the instrument tip 440 has sufficient flexibility and/or stiffness for the surgical procedure. Because the coating 444, and not the tip internal frame 442, directly contacts the patient's anatomy and because the coating 444 additionally satisfies technical requirements for the instrument tip 440, a cost-effective material can be a chosen for the tip internal frame 442 (e.g., lower cost bulk goods with less stringent technical requirements).

Covering the tip internal frame 442 (step 120) can include injection molding with a first coating 444, such as a metal, a metal alloy, a ceramic, a composite, a polymer, a plastic, an elastomer, and/or any other suitable material. The coating 444 can be chosen based on surgical objectives. For example, using a non-metallic coating 444 can provide lower friction between the instrument tip 440 and the patient's anatomy (e.g., the stroma of the cornea). The tip internal frame 442 can be used as an insert in the injection molding process. In other embodiments, other materials/industrial processing can be utilized to cover the tip internal frame 442 with the coating 444.

In some embodiments, the tip internal frame 442 can be solid. Thus, covering the tip internal frame 442 with the coating 444 (step 120) includes surrounding the tip internal frame 442 with the coating 444. In some embodiments, the tip internal frame 442 can include spaces that are permeable to the coating 444. Thus, covering the tip internal frame 442 with the coating 444 (step 120) can include both filling the spaces and surrounding the tip internal frame 442 with the coating 444.

The instrument tip 440 can be variously shaped in different embodiments, depending on, e.g., the instrument needs for different surgical procedures. For example, one or more sections of the instrument tip 440 can be straight, angular, curved, arcuate, etc.; include a hook, etc.; and/or define forceps, blades, scissors, etc. The tip internal frame 442 can define an internal structure of any shape needed for the instrument tip 440. The coating 444 can define the external surface of any shape needed for the instrument tip 440. An exemplary embodiment of an instrument tip 440 used in flapless refractive surgery is described herein. It is understood that the teachings of the present disclosure can be applied to various instruments used in different ophthalmic surgical procedures and other medical procedures.

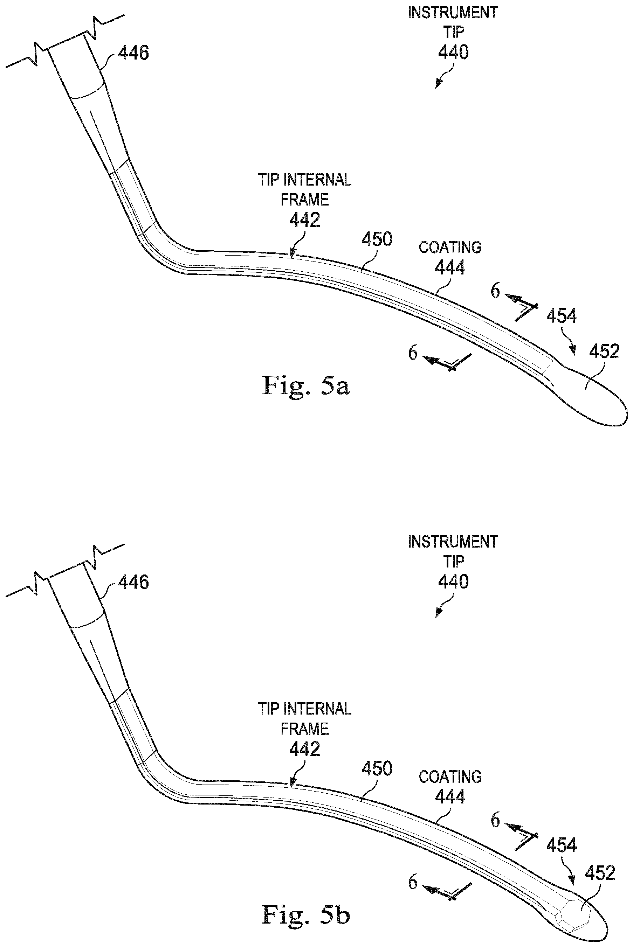

As shown in **FIGS. 4****,** **5a,** and **5b****,** the tip internal frame 442 can include a first section 446 and a second section 450. The second section 450 can be used by a surgeon to manipulate the patient's anatomy during the surgical procedure. The first section 446 can be generally linear and extend along a longitudinal axis 430 of the instrument body 420 **(****FIGS. 9a** and **9b****).** The second section 450 can extend obliquely from the first section 446. The second section 450 can be arcuately shaped. In some embodiments, the second section 450 of the tip internal frame 442 can be linearly shaped, and the coating 444 can cover the second section 450 of the tip internal frame 442 such that exterior profile of the second section 450 is arcuately shaped. In some embodiments, only the second section 450 is covered with the coating 444. In some embodiments, both the first and second sections 446 and 450 are covered with the coating 444. For different surgical instruments, the first and second sections 446 and 450 can be variously shaped. For example, the second section 450 can be angled, include a hook, etc.

The shape of the tip internal frame 442 can be similar to the desired exterior profile of the instrument tip 440. However, the shape of the tip internal frame 442 can be simpler than the final exterior profile. For example, one or more surfaces features (e.g., constant small edge radius at the distal end 454) of the exterior surface can be defined by the coating 444 without being replicated in the tip internal frame 442. In some embodiments, the coating 444 defines a smooth exterior profile of the instrument tip 400. In some embodiments, the coating 444 can define one or more surface features at the distal end 454. The surface features can include projections, recesses, grooves, ridges, striations, bumps, and/or other textural features. The surface features can modify the friction and/or contact feel between the instrument tip 440 and the patient's anatomy compared when the coating 444 defines a smooth exterior profile.

The tip internal frame 442 includes a proximal end 456 and a distal end 454. The distal end 454 of the tip internal frame 442 includes a flattened portion 452. The flattened portion 452 can be variously shaped in different embodiments, for example, as substantially circular, rectangular, elliptical, hexagonal, polygonal, a combination thereof, or any other profile. The flattened portion 452, covered with the coating 444, can have additional surface area for contact with the patient's anatomy compared to other portions of the instrument tip 440. The additional surface area can facilitate manipulation of, e.g., one or more layers of the patient's eye during the surgical procedure. A magnitude of a dimension (e.g., a radius or a width) of the flattened portion 452 in a direction perpendicular to the longitudinal axis 430 **(****FIGS. 9a** and **9b****)** can be greater than the magnitude of the dimension (e.g., the radius or the width) of the first and/or second sections 446 and 450. The coating 444 can define the flattened portion 452. For example, as shown in **FIG. 5a**, in some embodiments, the coating 444 defines the interior and the exterior of the flattened portion 452 such that the tip internal frame 442 does not extend through the flattened portion 452. As shown in FIG. 5b, in some embodiments, the tip internal frame 442 (e.g., the second section 450) extends through the flattened portion 452. The instrument tip 440 can have a length between approximately 1 mm and 50 mm, 10 mm and 40 mm, 20 mm and 40 mm, and 30 mm and 40 mm, in various embodiments. The instrument tip 440 can have a diameter between approximately 0.1 mm and 2 mm, 0.1 mm and 1 mm, 0.1 mm and 0.5 mm, and 0.1 mm and 0.3 mm, in various embodiments. The coating 444 can have a thickness at the distal end 454 of the instrument tip 440 between approximately 0.01 mm and 1 mm, 0.01 mm and 0.5 mm, 0.01 mm and 0.25 mm, and 0.01 mm and 0.15 mm, in various embodiments.

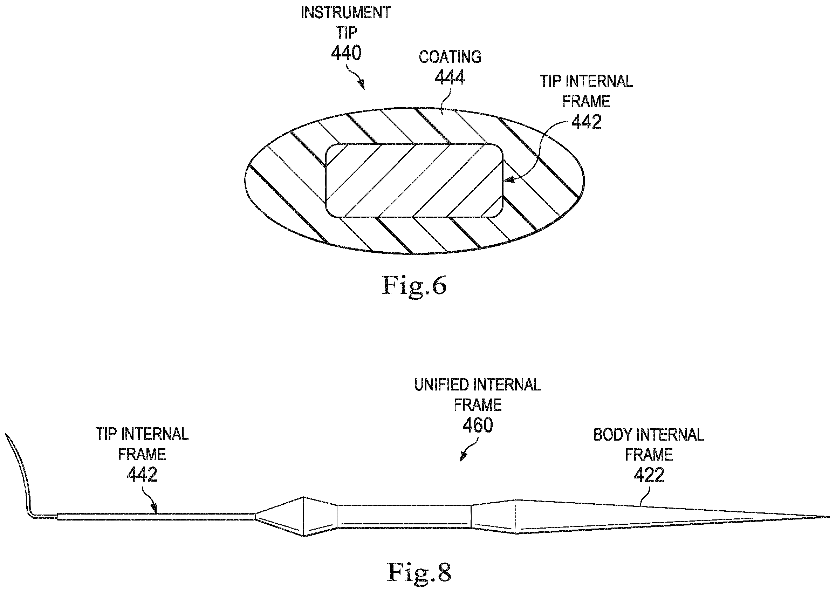

The tip internal frame 442 can have a cross-section in a plane perpendicular to the longitudinal axis 430 **(****FIGS. 9a** and **9b****)** that is shaped as a circle, rectangle, ellipse, polygon, a combination thereof, or any other profile. For example, as shown in **FIG. 6**, which is a cross-sectional view along line A-A of **FIGS. 5a** and **5b****,** the tip internal frame 442 has a cross-section that is rectangular with rounded corners. Similarly, the coating 444 can have a cross-section in the plane perpendicular to the longitudinal axis 430 **(****FIGS. 9a** and **9b****)** that is shaped as a circle, rectangle, ellipse, polygon, a combination thereof, or any other profile. For example, as shown in **FIG. 6**, the coating 444 has a cross-section that is elliptical. Thus, in some embodiments, as shown in **FIG. 6**, the cross-sectional profiles of the tip internal frame 442 and the coating 444 define different shapes. In some embodiments, the cross-sectional profiles of the tip internal frame 442 and the coating 444 define the same or similar shapes (e.g., both are elliptical, both are rectangular, etc.).

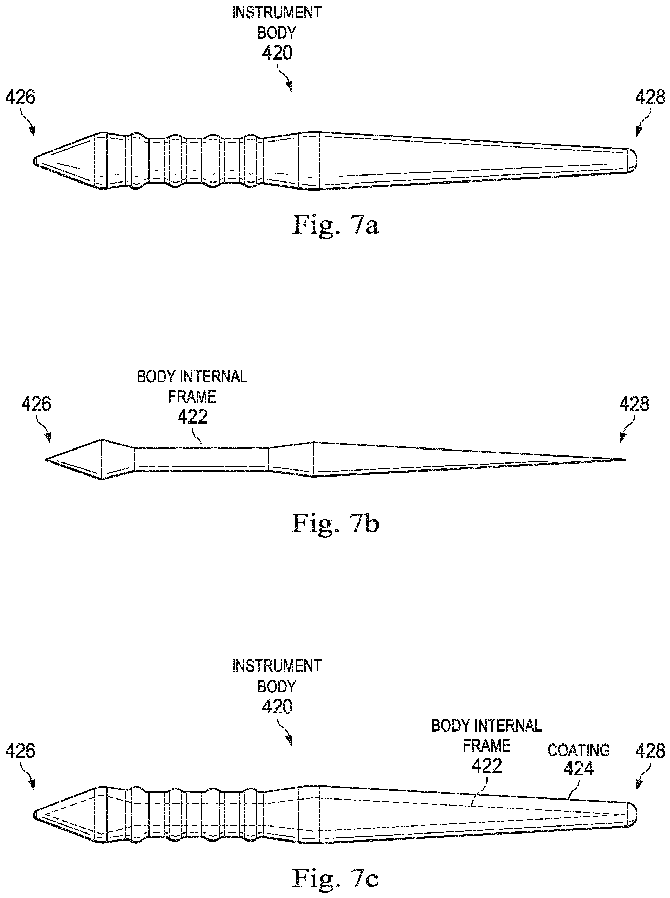

Referring again to **FIG. 1**, the method 100, at step 130, includes generating an instrument body. As shown in **FIGS. 7a**, **7b****,** **9a,** and **9b****,** the instrument body 420 is sized and shaped for grasping by a user (e.g., a surgeon) during a surgical procedure. The instrument body 420 can be a single component. For example, as shown in **FIG. 7a**, an instrument body 420 without a distinct internal frame or external coating can be utilized. The instrument body 420 of **FIG. 7a** can be hand formed or machine manufactured. For example, manufacturing the instrument body 320 can include turning (e.g., on a lathe), injection molding, milling, 3D printing, or any other suitable manufacturing method. In some embodiments, the instrument 420 can be a metallic component. However, according to the invention, the instrument body 420 is defined using the first coating 444. The coating 424 and/or coating 444 can be described as a molding material, such as the molding material used in an injection molding process. For example, the interior structure and the exterior surface of the instrument body 420 can be defined by the first coating 444. The instrument body 420 can be solid or hollow. In some embodiments, the instrument body 420 is generated using an injection molding process or other suitable materials/industrial process. According to the invention, covering the tip internal frame 442 with the first coating 444 (step 120) occurs substantially simultaneously (e.g., during the same processing step) as generating the instrument body 420 (step 130). The tip internal frame 442 can be used as an insert mold for injection molding. A thin layer of the first coating 444 can cover the tip internal frame 442 with the first coating 444 to define the exterior surface of the distal end 454, and the instrument body 420 can be molded as one piece with the first coating 444. The instrument 400 (**FIG. 9a**) can be formed thus formed in one embodiment. The method 100 can additionally include one or more finishing steps (e.g., polishing, laser marking, sterilizing, packaging, etc.).

**FIG. 2** illustrates a method 200 for manufacturing an ophthalmic surgical instrument with an instrument body and instrument tip each including an internal frame and an external coating, this method not being part of the claimed invention. The method 200, at step 210, can include generating an internal frame of the instrument tip. The method 200, at step 220, can further include covering the tip internal frame with an external coating to define an exterior surface. The tip internal frame 442 can be generated in a similar manner as described with respect to step 110 of method 100 (**FIG. 1**). For example, the tip internal frame 442 can be made of or include a metal, a metal alloy, a ceramic, a composite, a polymer, a plastic, an elastomer, and/or any other suitable material. The tip internal frame 442 can be covered with the external coating in a similar manner as described with respect to step 120 of method 100 (**FIG. 1**). For example, covering the tip internal frame 442 (step 220) can include injection molding with the coating, such as a metal, a metal alloy, a ceramic, a composite, a polymer, a plastic, an elastomer, and/or any other suitable material.

The method 200, at step 230, can include generating an internal frame for an instrument body. **FIGS. 7b**, **7c**, and **9b** can illustrate the body internal frame 422. The body internal frame 422 can define an internal structure of the instrument body 420. The method 200 can further include, at step 240 (**FIG. 2**), covering the body internal frame with a second coating to define an exterior surface of the instrument body. Steps 230 and 240, which are related to the body internal frame 422 and corresponding coating 424 of the instrument body, can be similar to steps 210 and 220 for the tip internal frame 442 and corresponding coating 444. For example, the body internal frame 422 can be made of or include a second material, such as a metal, a metal alloy, a ceramic, a composite, a polymer, a plastic, an elastomer, and/or any other suitable material. The same material or different materials can be used for the body internal frame 422 and the tip internal frame 442. Covering the body internal frame 422 (step 240) can include injection molding with a second coating 424 (**FIGS. 7c** and **9b**), such as a metal, a metal alloy, a ceramic, a composite, a polymer, a plastic, an elastomer, and/or any other suitable material. The first coating 444 and the second coating 424 can be the same or different.

As shown in **FIGS. 7b**, **7c**, and **9b**, the shape of the body internal frame 422 can be similar to the desired exterior profile of the instrument body 420. However, the shape of the body internal frame 422 can be simpler than the final exterior profile. For example, one or more surface features defined by the coating 424 to enhance the user's grip of the instrument body 420 can be omitted from the body internal frame 422. The coating 424 can define a textured surface (e.g., roughened, knurled, projections/recesses, tapers, other surface features, and/or combinations thereof) on the instrument body 420.

Referring again to **FIG. 2**, the method 200, at step 250, can include bringing the instrument tip and the instrument body into engagement. In some embodiments, the tip internal frame 442 can be engaged with the body internal frame 422. For example, the proximal end 456 of the tip internal frame 442 can be mechanically coupled to the distal end 426 of the body internal frame 422 via an attachment mechanism 448 (**FIG. 4**). The attachment mechanism 448 can be disposed at the proximal end 456 of the tip internal frame 442. The attachment mechanism 448 can include any suitable mechanical configuration to allow for the instrument tip 440 to be joined to the instrument body 420. The attachment mechanism 448 can be omitted in embodiments in which the tip internal frame 442 and body internal frame 422 are not separate components. In **FIG. 4**, the attachment mechanism 448 can have a radius less than the first and/or sections 446 and 450. For example, the attachment mechanism 448 can include external threads that are configured to mate with corresponding internal threads at the distal end 426 of the instrument body 420. In some embodiments, the tip internal frame 442 is configured to be press fit, slip fit, compression fit, interference fit, or otherwise engagingly fit with the instrument body 420. In some embodiments, the connection between instrument tip 440 and the instrument body 420 is form- and/or force-closed. For example, in an injection molding processing, tip internal frame 442 can be inserted into an injection molding machine, and the proximal end 456 of the tip internal frame 442 can be over molded. In some embodiments, the tip internal frame 442 can be engaged with the coating 424 of the instrument body 420 or the body internal frame 422 can be engaged with the coating 444 of the instrument tip 440. In some embodiments, an adhesive can be used to engage the instrument body 420 and the instrument tip 440. The instrument 400 (**FIG. 9b**) is formed when the instrument body 420 and the instrument tip 440 are brought into engagement.

**FIG. 3** illustrates a method 300 for manufacturing an ophthalmic surgical instrument with a unified internal frame, this method not being part of the claimed invention. The method 300, at step 310, can include generating a unified internal frame of the instrument tip and the instrument body. The method 300, at step 320, can further include covering the unified internal frame with an external coating to define an exterior surface. As shown in **FIG. 8**, the tip internal frame 442 and the body internal frame 422 can comprise a unitary component. The unified internal frame 460 can be generated in a similar manner as described with respect to step 110 of method 100 **(****FIG. 1****).** For example, the unified internal frame 460 can be made of or include a metal, a metal alloy, a ceramic, a composite, a polymer, a plastic, an elastomer, and/or any other suitable material. The unified internal frame 460 can be covered with the external coating in a similar manner as described with respect to step 120 of method 100 **(****FIG. 1****).** For example, covering the unified internal frame 460 (step 320) can include injection molding with the coating, such as a metal, a metal alloy, a ceramic, a composite, a polymer, a plastic, an elastomer, and/or any other suitable material. As shown in **FIGS. 8** and **9b****,** the shape of the unified internal frame 460 can be similar to the desired exterior profile of the instrument body 420 and the instrument tip 440. However, the shape of the body internal frame 422 can be simpler than the final exterior profile. For example, one or more features defined by the coating (such as complex shapes of the instrument tip 440, surface features of the instrument body 420, etc.) can be omitted from the unified internal frame 460. The instrument 400 **(****FIG. 9b****)** is formed when the unified internal frame 460 is covered by the external coating. The method 300 can additionally include one or more finishing steps (e.g., polishing, laser marking, sterilizing, packaging, etc.).

Suitable materials/industrial processing, including blanking, molding, casting, machining, cutting, etc., can be used to generate the tip internal frame 442, the body internal frame 422, and/or the unified internal frame 460, and/or covering an internal frame with an external coating. For example, blow molding, injection molding, thermoforming, centrifugal casting, investment casting, permanent mold casting, sand casting, shell mold casting, milling, turning, forming, shearing, punching, laser beam cutting, plasma cutting, water jet cutting, stereolithography, fused deposition modeling, selective laser sintering, direct metal laser sintering, 3D printing, extrusion, electric discharge machining, electrochemical machining, electroforming, bending, roll forming, spinning, deep drawing, stretch forming, among others, can be utilized.

Embodiments as described herein can provide devices, systems, and methods that facilitate the manufacture of ophthalmic surgical instruments with complex shapes while satisfying necessary technical requirements for flexibility and/or stiffness. The devices, systems, and methods described herein can be used with any surgical instrument. The examples provided above are exemplary only and are not intended to be limiting. One skilled in the art may readily devise other systems consistent with the disclosed embodiments which are intended to be within the scope of this disclosure. As such, the application is limited only by the following claims.

## Claims

1. A method of manufacturing an ophthalmic surgical instrument (400), comprising:
generating an internal frame (442) defining an internal structure of an instrument tip (440), the instrument tip (440) comprising:
a proximal end (456)
a distal end (454);
a linear first section (446);
an arcuate second section (450) extending obliquely from the first section (446); and
a flattened portion (452) at the distal end (454);
**characterized in that** the method further comprises:
generating an instrument body (420) using a coating, the instrument body comprising:
a longitudinal axis (430); a proximal end (426) and a distal end (428); and
an external surface of the instrument body (420) including surface features; and
covering the internal frame (442) with the coating (444) to define an exterior surface of the instrument tip (440) simultaneously as generating the instrument body,
wherein the cross-sectional profiles of the internal frame (442) and coating (444) in a plane perpendicular to the longitudinal axis (430) of the instrument body define different shapes along at least a portion of the longitudinal axis.

2. The method of claim 1,
wherein covering the internal frame (442) with the coating (444) comprises injection molding to cover the internal frame with at least one of a metal, a metal alloy, a ceramic, a composite, a polymer, a plastic, and an elastomer.

3. The method of claim 1, wherein: generating the internal frame (442) includes at least one of molding, casting, machining, and cutting of a first material.

4. The method of claim 3, wherein: the first material is at least one of a metal, a metal alloy, a ceramic, a composite, a polymer, a plastic, and an elastomer.

5. The method of claim 1, further comprising: engaging the proximal end (456) of the instrument tip and the distal end (426) of the instrument body.

6. The method of claim 1, the method further comprising at least one of:
(i) covering at least a portion of the internal frame (442) with the coating (444) includes covering the distal end (454) and the arcuate second section (450) with the first coating (444); and
(ii) covering the internal frame (442) with the coating (444) includes defining a flattened portion (452) at a distal end of the instrument tip.

7. An ophthalmic surgical instrument (400) manufactured according to the method of claim 1, the ophthalmic surgical instrument (400) comprising:
an instrument body (420) comprising:
a proximal end (428);
a distal end (426); and
a longitudinal axis (430); and
an instrument tip (440) disposed at the distal end of the instrument body, the instrument tip comprising:
a first internal frame (442) including a first section (446) linearly extending along a longitudinal axis of the instrument body and a second section (450) extending obliquely from the first section (446), the second section (450) being arcuately shaped; and
a first coating (444) covering the first internal frame (442) to define an exterior surface of the instrument tip (440).

8. The ophthalmic surgical instrument of claim 7, wherein cross- sectional profiles of the first internal frame (442) and the first coating (444) in a plane perpendicular to the longitudinal axis (430) of the instrument body (420) define different shapes.

9. The ophthalmic surgical instrument of claim 7, wherein the instrument tip (440) includes a flattened portion (452) at a distal end (454).

10. The ophthalmic surgical instrument of claim 7, wherein the instrument body (420) comprises the first coating (444).

11. The ophthalmic surgical instrument of claim 7, wherein the instrument body (420) comprises a second internal frame (422) and a second coating (424) covering the second internal frame.

12. The ophthalmic surgical instrument of claim 11, wherein the first internal frame (424) and second internal frame (422) comprise a unitary component.

13. The ophthalmic surgical instrument of claim 11, wherein the first (442) and second (422) internal frames, and the first (444) and second coatings (424) comprise at least one of a metal, a metal alloy, a ceramic, a composite, a polymer, a plastic, and an elastomer.

## Patentansprüche

1. Verfahren zur Herstellung eines ophthalmischchirurgischen Instruments (400), umfassend:
Generieren eines Innenrahmens (442), der eine Innenstruktur einer Instrumentenspitze (440) definiert, wobei die Instrumentenspitze (440) umfasst:
ein proximales Ende (456);
ein distales Ende (454);
einen linearen ersten Abschnitt (446);
einen bogenförmigen zweiten Abschnitt (450), der sich schräg von dem ersten Abschnitt (446) erstreckt; und
einen abgeflachten Anteil (452) an dem distalen Ende (454);
**dadurch gekennzeichnet, dass** das Verfahren ferner umfasst:
Generieren eines Instrumentenkörpers (420) unter Verwendung einer Beschichtung, wobei der Instrumentenkörper umfasst:
eine Längsachse (430); ein proximales Ende (426) und ein distales Ende (428); und
eine Außenoberfläche des Instrumentenkörpers (420) einschließlich Oberflächenmerkmalen; und
Bedecken des Innenrahmens (442) mit der Beschichtung (444), um eine Außenoberfläche der Instrumentenspitze (440) gleichzeitig mit dem Generieren des Instrumentenkörpers zu definieren, wobei die Querschnittprofile des Innenrahmens (442) und der Beschichtung (444) in einer Ebene senkrecht zu der Längsachse (430) des Instrumentenkörpers unterschiedliche Gestalten entlang mindestens eines Anteils der Längsachse definieren.

2. Verfahren nach Anspruch 1,
wobei das Bedecken des Innenrahmens (442) mit der Beschichtung (444) Spritzgießen umfasst, um den Innenrahmen mit mindestens einem von einem Metall, einer Metalllegierung, einer Keramik, einem Verbundmaterial, einem Polymer, einem Kunststoff und einem Elastomer zu bedecken.

3. Verfahren nach Anspruch 1, wobei: Generieren des Innenrahmens (442) mindestens eines von Formen, Gießen, maschinellem Bearbeiten und Fräsen aus einem ersten Material einschließt.

4. Verfahren nach Anspruch 3, wobei: das erste Material mindestens eines von einem Metall, einer Metalllegierung, einer Keramik, einem Verbundmaterial, einem Polymer, einem Kunststoff und einem Elastomer ist.

5. Verfahren nach Anspruch 1, des Weiteren umfassend: In-Eingriff-bringen des proximalen Endes (456) der Instrumentenspitze und des distalen Endes (426) des Instrumentenkörpers.

6. Verfahren nach Anspruch 1, wobei das Verfahren des Weiteren mindestens eines der folgenden umfasst:
(i) Bedecken von mindestens einem Anteil des Innenrahmens (442) mit der Beschichtung (444) schließt Bedecken des distalen Endes (454) und des bogenförmigen zweiten Abschnitts (450) mit der ersten Beschichtung (444) ein; und
(ii) Bedecken des Innenrahmens (442) mit der Beschichtung (444) schließt Definieren eines abgeflachten Anteils (452) an einem distalen Ende der Instrumentenspitze ein.

7. Ophthalmisch-chirurgisches Instrument (400), das gemäß dem Verfahren von Anspruch 1 hergestellt ist, wobei das ophthalmisch-chirurgische Instrument (400) umfasst:
einen Instrumentenkörper (420), umfassend:
ein proximales Ende (428);
ein distales Ende (426); und
eine Längsachse (430); und
eine Instrumentenspitze (440), die an dem distalen Ende des Instrumentenkörpers angeordnet ist, wobei die Instrumentenspitze umfasst:
einen ersten Innenrahmen (442), der einen ersten Abschnitt (446), der sich linear entlang einer Längsachse des Instrumentenkörpers erstreckt, und einen zweiten Abschnitt (450) einschließt, der sich schräg von dem ersten Abschnitt (446) erstreckt, wobei der zweite Abschnitt (450) bogenförmig gestaltet ist; und
eine erste Beschichtung (444), die den ersten Innenrahmen (442) bedeckt, um eine Außenoberfläche der Instrumentenspitze (440) zu definieren.

8. Ophthalmisch-chirurgisches Instrument nach Anspruch 7, wobei Querschnittprofile des ersten Innenrahmens (442) und der ersten Beschichtung (444) in einer Ebene senkrecht zu der Längsachse (430) des Instrumentenkörpers (420) unterschiedliche Gestalten definieren.

9. Ophthalmisch-chirurgisches Instrument nach Anspruch 7, wobei die Instrumentenspitze (440) einen abgeflachten Anteil (452) an dem distalen Ende (454) einschließt.

10. Ophthalmisch-chirurgisches Instrument nach Anspruch 7, wobei der Instrumentenkörper (420) die erste Beschichtung (444) umfasst.

11. Ophthalmisch-chirurgisches Instrument nach Anspruch 7, wobei der Instrumentenkörper (420) einen zweiten Innenrahmen (422) und eine zweite Beschichtung (424) umfasst, die den zweiten Innenrahmen bedeckt.

12. Ophthalmisch-chirurgisches Instrument nach Anspruch 11, wobei der erste Innenrahmen (424) und der zweite Innenrahmen (422) eine einteilige Komponente umfassen.

13. Ophthalmisch-chirurgisches Instrument nach Anspruch 11, wobei der erste (442) und der zweite (422) Innenrahmen und die erste (444) und zweite Beschichtung (424) mindestens eines von einem Metall, einer Metalllegierung, einer Keramik, einem Verbund, einem Polymer, einem Kunststoff und einem Elastomer umfassen.

## Revendications

1. Procédé de fabrication d'un instrument chirurgical ophtalmique (400), comprenant :
la génération d'une structure interne (442) définissant une structure interne d'une pointe (440) d'instrument, la pointe (440) d'instrument comprenant :
une extrémité proximale (456) ;
une extrémité distale (454) ;
une première section linéaire (446) ;
une seconde section arquée (450) s'étendant obliquement depuis la première section (446) ; et
une partie aplatie (452) au niveau de l'extrémité distale (454) ;
**caractérisé en ce que** le procédé comprend en outre :
la génération d'un corps (420) d'instrument à l'aide d'un revêtement, le corps d'instrument comprenant :
un axe longitudinal (430) ; une extrémité proximale (426) et une extrémité distale (428) ; et
une surface externe du corps (420) d'instrument comportant des caractéristiques de surface ; et
le recouvrement de la structure interne (442) avec le revêtement (444) pour définir une surface extérieure de la pointe (440) d'instrument simultanément à la génération du corps d'instrument,
dans lequel les profils en coupe transversale de la structure interne (442) et du revêtement (444) dans un plan perpendiculaire à l'axe longitudinal (430) du corps d'instrument définissent des formes différentes le long d'au moins une partie de l'axe longitudinal.

2. Procédé selon la revendication 1,
dans lequel le recouvrement de la structure interne (442) avec le revêtement (444) comprend le moulage par injection pour recouvrir la structure interne avec au moins un parmi un métal, un alliage de métal, une céramique, un composite, un polymère, un plastique et un élastomère.

3. Procédé selon la revendication 1, dans lequel : la génération de la structure interne (442) comprend au moins un parmi un moulage, un coulage, un usinage et un découpage d'un premier matériau.

4. Procédé selon la revendication 3, dans lequel : le premier matériau est au moins un parmi un métal, un alliage de métal, une céramique, un composite, un polymère, un plastique et un élastomère.

5. Procédé selon la revendication 1, comprenant en outre : la mise en prise de l'extrémité proximale (456) de la pointe d'instrument et de l'extrémité distale (426) du corps d'instrument.

6. Procédé selon la revendication 1, le procédé comprenant en outre au moins un des éléments suivants :
(i) le recouvrement d'au moins une partie de la structure interne (442) avec le revêtement (444) comprend le recouvrement de l'extrémité distale (454) et de la seconde section arquée (450) avec le premier revêtement (444) ; et
(ii) le recouvrement de la structure interne (442) avec le revêtement (444) comprend la définition d'une partie aplatie (452) au niveau d'une extrémité distale de la pointe d'instrument.

7. Instrument chirurgical ophtalmique (400) fabriqué selon le procédé selon la revendication 1, l'instrument chirurgical ophtalmique (400) comprenant :
un corps (420) d'instrument comprenant :
une extrémité proximale (428) ;
une extrémité distale (426) ; et
un axe longitudinal (430) ; et
une pointe (440) d'instrument disposée au niveau de l'extrémité distale du corps d'instrument, la pointe d'instrument comprenant :
une première structure interne (442) comprenant une première section (446) s'étendant linéairement le long d'un axe longitudinal du corps d'instrument et une seconde section (450) s'étendant obliquement depuis la première section (446), la seconde section (450) étant de forme arquée ; et
un premier revêtement (444) recouvrant la première structure interne (442) pour définir une surface extérieure de la pointe (440) d'instrument.

8. Instrument chirurgical ophtalmique selon la revendication 7, dans lequel les profils en coupe transversale de la première structure interne (442) et du premier revêtement (444) dans un plan perpendiculaire à l'axe longitudinal (430) du corps (420) d'instrument définissent des formes différentes.

9. Instrument chirurgical ophtalmique selon la revendication 7, dans lequel la pointe (440) d'instrument comprend une partie aplatie (452) au niveau d'une extrémité distale (454).

10. Instrument chirurgical ophtalmique selon la revendication 7, dans lequel le corps (420) d'instrument comprend le premier revêtement (444).

11. Instrument chirurgical ophtalmique selon la revendication 7, dans lequel le corps (420) d'instrument comprend une seconde structure interne (422) et un second revêtement (424) recouvrant la seconde structure interne.

12. Instrument chirurgical ophtalmique selon la revendication 11, dans lequel la première structure interne (424) et la seconde structure interne (422) comprennent un élément monobloc.

13. Instrument chirurgical ophtalmique selon la revendication 11, dans lequel les première (442) et seconde (422) structures internes, et les premier (444) et second (424) revêtements comprennent au moins un parmi un métal, un alliage de métal, une céramique, un composite, un polymère, un plastique et un élastomère.
